# EUROPÄISCHE PATENTSCHRIFT

(11) **EP 0 784 047 B1**
(45) Veröffentlichungstag und Bekanntmachung des Hinweises auf die Patenterteilung: **08.12.1999**
(21) Anmeldenummer: 97100030.2
(22) Anmeldetag: 03.01.1997
(51) Int. Cl.: C07C 59/06, C07C 51/42

(54) **Verfahren zur Darstellung einer besonders reinen Glykolsäure**
Process for producing highly pure glycolic acid
Procédé de production d'acide glycolique de haute purété

(30) Priorität: 10.01.1996 DE 19600620
(43) Veröffentlichungstag der Anmeldung: 16.07.1997
(73) Patentinhaber: Clariant GmbH, 65929 Frankfurt am Main (DE)
(72) Erfinder: Ebmeyer, Frank, Dr., 86153 Augsburg (DE); Häberlein, Harald, Dr., verstorben (DE); Mohn, Holger, Dr., 63571 Gelnhausen-Hailer (DE)

(56) Entgegenhaltungen:
- AT-B- 399 721
- DE-A- 1 959 897
- DE-A- 3 812 183
- FR-A- 2 675 709
- US-A- 4 781 809
- US-A- 5 057 197

## Beschreibung

Die Erfindung beschreibt ein Verfahren zur Darstellung einer besonders reinen Glykolsäure, wobei die Reinheit sich auf die weitestgehende Freiheit von Verunreinigungen wie Alkalimetallchloriden, organischen Säuren, Aldehyden, Stickstoffverbindungen und Salzen jeglicher Art bezieht. Eine solche Glykolsäure hoher Qualität findet in der pharmazeutischen Industrie sowie in der Kosmetikindustrie Verwendung.

Diese Reinheit kann nicht durch einfache Destillation oder Kristallisation erreicht werden. Eine Destillation scheidet aus, da sich Glykolsäure hierbei zersetzt. Bei der Kristallisation erfolgt keine hinreichende Reinigung, da obige Verunreinigungen wie Salze und organische Säuren mit auskristallisieren oder der Glykolsäure anhaften bleiben.

Bekannt ist die Darstellung von Glykolsäure durch Verseifung von Chloressigsäure mit Alkalimetallhydroxiden, wobei eine weitgehende Entfernung des anfallenden Natriumchlorids durch Filtration und anschließende Fällung des in der Lösung verbleibenden Natriumchlorids durch Zugabe von Methylethylketon oder Methylisobutylketon erfolgen kann. Das organische Lösungsmittel wird anschließend abdestilliert (DE-A-28 12 682, DE-A-28 12 683). Der Nachteil dieses Verfahrens besteht in der Verwendung eines organischen Lösungsmittels sowie darin, daß sich organische Verunreinigungen auf diese Weise nicht entfernen lassen.

Weiterhin bekannt ist die Herstellung von Glykolsäure durch Carbonylierung von Formaldehyd (WO 92/05138). Die als Verunreinigungen anfallenden Komponenten, insbesondere Formaldehyd, Ameisensäure und Methoxyessigsäure verbleiben hierbei jedoch zu erheblichen Teilen im Produkt. Zur Entfernung von Salzen sind auch Verfahren unter Verwendung von Ionenaustauschern durchführbar. Die notwendige Regenierung der Ionenaustauscher macht diese Verfahren jedoch sehr umständlich und erzeugt zudem Abwässer aus der Regenerierung.

Daneben ist die Herstellung von Glykolsäure durch elektrochemische Reduktion von Oxalsäure beschrieben (DE 194 038). Die Gewinnung eines besonders reinen Produktes ist auch bei diesem Verfahren schwierig, da das sich chemisch sehr ähnlich verhaltende Nebenprodukt Glyoxylsäure wie auch nicht umgesetzte Oxalsäure die Gewinnung von hochreinen Qualitäten erschweren.

Weiterhin ist die Darstellung von Glykolsäure durch Hydrolyse des Glykonitrils, welches durch Umsetzung von Formaldehyd mit Blausäure gewonnen wird, beschrieben (DE-A-1254615). Hierbei sind die während der Hydrolyse anfallenden Stickstoffverbindungen, insbesondere Ammoniumsalze, sehr schwer abzutrennen, was die Gewinnung einer hochreinen Qualität erschwert.

Aufgabe der vorliegenden Erfindung war somit, ein im technischen Maßstab realisierbares Verfahren zur Darstellung einer besonders reinen Glykolsäure zur Verfügung zu stellen, das die vorher beschriebenen Nachteile nicht aufweist.

Überraschenderweise wurde nun gefunden, daß man eine besonders reine Glykolsäure durch Verseifung von Chloressigsäure mit einem Überschuß an Alkalimetallhydroxid unter Abfiltration des entstandenen Alkalichlorids erhält, wenn man anschließend die 20 bis 70 gew.%ige Glykolsäurelösung bei 20 bis 40°C und einer Zellspannung von 0,5 bis 2,5 V pro Zellenpaar einer Elektrodialyse unterwirft.

Die Verseifung erfolgt mit einem Überschuß an Alkalimetallhydroxid bis 10, vorzugsweise von 2 bis 8 Gew.-%. Von den Alkalimetallhydroxiden haben sich NaOH oder KOH als besonders geeignet erwiesen. Die Verseifung wird vorteilhafterweise bei einer Temperatur von 100 bis 160°C, insbesondere von 110 bis 150°C sowie bei einem Überdruck von 0 bis 10, insbesondere von 0 bis 5 bar durchgeführt. Nach der Verseifung verbleiben im Verseifungsprodukt lediglich Spuren von Chloressigsäuren, danach wird, nach Abfiltration des entstandenen Alkalimetallchlorids, mit einer Elektrodialyse eine weitestgehende Entsalzung der Lösung sowie eine weitere Konzentrationsabnahme der Chloressigsäuren erzielt.

Zwischen Verseifung und Elektrodialyse ist die Filtration zwischengeschaltet, über die der größte Teil des Natriumchlorids bereits abgetrennt wird. Hierdurch ist es möglich, das in der Elektrodialyse anfallende Salzkonzentrat in die Verseifung zurückzuführen. Es entsteht somit kein verfahrensbedingtes Abwasser.

Die weitere Reinigung durch Elektrodialyse wird dadurch möglich, daß in der Verseifung ein Überschuß an Alkalimetallhydroxid verwendet wird, welcher im Verseifungsprozeß verbleibt. Da Chloressigsäuren stärkeren Säuren sind als Glykolsäure, liegen diese folglich im Verseifungsprodukt ionisch als Alkalimetallcarboxylate vor und können mittels Elektrodialyse ausgeschleust werden.

Die Elektrodialyse wird bei einer Temperatur von 20 bis 40°C durchgeführt. Die Glykolsäurekonzentration im Diluat beträgt dabei 20 bis 70, insbesondere 40 bis 60 Gew.-%. Als geeignet hat sich eine angelegte Zellspannung von 0,5 bis 2,5, insbesondere von 1 bis 2 V erwiesen.

Das erfindungsgemäße Verfahren erlaubt eine optimale Entfernung von Natriumchlorid und Chloressigsäure, wie es zur Darstellung einer besonders reinen Glykolsäure notwendig ist. Andere organische Säuren, Stickstoffverbindungen, Aldehyde und Salze entstehen hierbei nicht und wirken sich somit nicht auf die Qualität des Produktes aus.

Beispiel 1 zeigt eine Vorgehensweise nach dem erfindungsgemäßen Verfahren, bei der im Produkt die Verunreinigungen Chloressigsäure und Dichloressigsäure innerhalb einer Nachweisgrenze von 10 ppm nicht mehr nachweisbar sind.

Nach der Elektrodialyse wird etwas Wasser aus der Lösung destillativ entfernt und die Glykolsäurekonzentration auf den gewünschten Wert, für gewöhnlich 70 Gew.-%, eingestellt.

Die Erfindung wird durch die nachstehenden Beispiele erläutert, bei denen die Elektrodialyse unter folgenden Bedingungen durchgeführt wird:

Es kommt eine Laborapparatur der Firma Berghof (Bel 2) mit einem Membranstapel, bestückt mit 10 parallel angeordneten Zellenpaaren, bestehend aus Anionen- und Kationenaustauschermembranen (Hersteller Asahi Glass Co. LTD), zum Einsatz. Die effektive Membranfläche beträgt 37 cm². Die Elektroden bestehen aus platiniertem Titan. Es wird eine elektrische Spannung von 1,5 V pro Zellenpaar angelegt und die Membranen mit ca. 5 cm/s überströmt. Die Apparatur besitzt 3 Kreislaufströme. Der Diluatkreisstrom enthält die zu entsalzende Glykolsäurelösung. Der wässerige Konzentratstrom dient zur Aufnahme des Salzes. Die Elektrodenspüllösung (Na₂SO₄, 0,2 Gew.-%ig) verhindert ungewollte Elektrodenreaktionen wie beispielsweise die anodische Cl₂-Entwicklung. Die Versuchstemperatur liegt bei 20 bis 40°C. Alle Prozentangaben in den Beispielen sind als Gew.-% zu verstehen.

### Beispiele:

### Beispiel 1:

1,10 kg Chloressigsäure mit einem Dichloressigsäuregehalt von 300 ppm werden mit 1,00 kg 50 %iger Natronlauge (7,5 % Überschuß) versetzt und 70 h auf 115°C erhitzt. Danach werden 0,52 kg Natriumchlorid durch Filtration und 0,10 kg Wasser durch Destillation abgetrennt. Es verbleiben 1,41 kg 55 %ige wässerige Glykolsäure, welche 11 % Natriumchlorid und weniger als 100 ppm Chloressigsäure enthält. Diese Lösung wird der Elektrodialyse diluatseitig zugeführt, wobei auf der Konzentratseite 1,41 kg 0,25 %ige Natriumchloridlösung vorgelegt werden. Über die Versuchsdauer von 30 h wird die Spannung konstant auf 15 V gehalten. Der elektrische Strom liegt durchschnittlich bei ca. 0,6 A. Die Leitfähigkeit der Diluatlösung sinkt von 18 auf 5 mS/cm und steigt im Konzentrat von 5 auf 110 mS/cm. Man erhält 1,21 kg wässerige Glykolsäurelösung mit einem Säuregehalt von 52 % und Chloressigsäure- bzw. Dichloressigsäuregehalten von weniger als 10 ppm. Aus dieser Lösung wird durch Entfernung von 0,31 kg Wasser eine 70 %ige Glykolsäurelösung hergestellt. Der Gehalt an Chlor- und Dichloressigsäure beträgt weniger als 10 ppm, der Natriumchloridgehalt liegt bei 0,02 %.

### Beispiel 2:

1250 kg 80 %ige wässerige Chloressigsäurelösung mit einem Dichloressigsäuregehalt von 240 ppm werden mit 880 kg Natronlauge (4 % Überschuß) versetzt und 20 h in einem Reaktor bei einem Druck von 1,8 bar auf 145°C erhitzt. 476 kg Natriumchlorid werden abfiltriert und 360 kg Wasser abdestilliert. Es verbleiben 1282 kg 56 %ige Glykolsäurelösung, die 13 % Natriumchlorid und 500 ppm Chloressigsäuren enthält. 4,3 kg dieser Lösung werden der Elektrodialyse diluatseitig zugeführt, wobei auf der Konzentratseite 5 kg 0,25 %ige Natriumchloridlösung vorgelegt werden. Über die Versuchsdauer von 54 h wird die Spannung konstant auf 15 V gehalten. Der elektrische Strom liegt durchschnittlich bei ca. 0,6 A. Die Leitfähigkeit der Diluatlösung sinkt von 20 auf 4 mS/cm und steigt im Konzentrat von 5 auf 120 mS/cm. Man erhält 3,1 kg wässerige Glykolsäurelösung mit einem Säuregehalt von 57,3 %, 0,016 % NaCl und einem Gehalt an Chloressigsäuren von 380 ppm. Aus dieser Lösung wird durch Entfernung von 0,59 kg Wasser eine 70 %ige Glykolsäurelösung hergestellt.

### Beispiel 3:

Man verfährt wie in Beispiel 2, verwendet jedoch lediglich 868 kg 50 %ige Natronlauge (2,5 % Überschuß). Man erhält nach Abfiltrieren des Salzes eine Lösung, die 57 % Glykolsäure sowie 0,32 % Chloressigsäuren und 11,3 % NaCl enthält. 4 kg dieser Lösung werden der Elektrodialyse so diluatseitig zugeführt, wobei auf der Konzentratseite 4 kg 0,25 %ige Natriumchloridlösung vorgelegt werden. Über die Versuchsdauer von 48 h wird die Spannung konstant auf 15 V gehalten. Der elektrische Strom liegt durchschnittlich bei ca. 0,6 A. Die Leitfähigkeit der Diluatlösung sinkt von 31 auf 3 mS/cm und steigt im Konzentrat von 5 auf 115 mS/cm. Man erhält 2,9 kg wässerige Glykolsäurelösung mit einem Säuregehalt von 50,7 %, 0,004 % NaCl und einem Gehalt an Chloressigsäure von 0,13 %. Aus dieser Lösung wird durch Entfernung von 0,76 kg Wasser eine 70 %ige Glykolsäurelösung hergestellt. Der Gehalt an Chloressigsäuren beträgt 1300 ppm, der Natriumchloridgehalt liegt bei 0,004 %.

### Beispiel 4:

Man verfährt wie in Beispiel 2, verwendet jedoch eine 80 %ige wässerige Chloressigsäurelösung mit einem Dichloressigsäuregehalt von 0,25 %. Man erhält nach Abfiltrieren des Salzes eine Lösung, die 56 % Glykolsäure, 560 ppm Chloressigsäuren und 13 % NaCl enthält. 4 kg dieser Lösung werden der Elektrodialyse diluatseitig zugeführt, wobei auf der Konzentratseite 4 kg 0,25 %ige Natriumchloridlösung vorgelegt werden. Über die Versuchsdauer von 50 h wird die Spannung konstant auf 15 V gehalten. Der elektrische Strom liegt durchschnittlich bei ca. 0,6 A. Die Leitfähigkeit der Diluatlösung sinkt von 21 auf 3 mS/cm und steigt im Konzentrat von 4 auf 120 mS/cm. Man erhält 3 kg wässerige Glykolsäurelösung mit einem Säuregehalt von 59,5 % mit 0,012 % NaCl und einem Gehalt an Chloressigsäuren von 320 ppm. Aus dieser Lösung wird durch Entfernung von 0,42 kg Wasser eine 70 %ige Glykolsäurelösung hergestellt. Der Gehalt an Chloressigsäure beträgt 220 ppm, der Natriumchloridgehalt liegt bei 0,012 %.

## Patentansprüche

1. Verfahren zur Darstellung einer besonders reinen Glykolsäure durch Verseifung von Chloressigsäure mit einem Überschuß an Alkalimetallhydroxid unter Abfiltration des entstandenen Alkalichlorids, dadurch gekennzeichnet, daß man anschließend die 20 bis 70 gew.%ige Glykolsäurelösung bei 20 bis 40°C und einer Zellspannung von 0,5 bis 2,5 V pro Zellenpaar einer Elektrodialyse unterwirft.

2. Verfahren nach Anspruch 1 dadurch gekennzeichnet, daß die Glykolsäurekonzentration im Diluat 40 bis 60 Gew.-% beträgt.

3. Verfahren nach Anspruch 1, dadurch gekennzeichnet, daß die angelegte Zellspannung bei der Elektrodialyse 1 bis 2 V pro Zellenpaar beträgt.

## Claims

1. A process for preparing a particularly pure glycolic acid by saponification of chloroacetic acid with an excess of alkali metal hydroxide, with the resulting alkali metal chloride being filtered off, which comprises subsequently subjecting the 20 to 70% strength by weight glycolic acid solution to an electrodialysis at 20 to 40°C and a cell voltage of 0.5 to 2.5 V per cell pair.

2. The process as claimed in claim 1, wherein the glycolic acid concentration in the diluate is 40 to 60% by weight.

3. The process as claimed in claim 1, wherein the applied cell voltage in the electrodialysis is 1 to 2 V per cell pair.

## Revendications

1. Procédé de production d'acide glycolique particulièrement pur par saponification d'acide chloroacétique avec un excès d'hydroxyde de métal alcalin par filtration du chlorure de métal alcalin produit, caractérisé en ce qu'on réalise ensuite une électrodialyse de la solution d'acide glycolique à 20 à 70% en poids à 20 à 40°C et sous une tension de cellule de 0,5 à 2,5 V par paire de cellules.

2. Procédé selon la revendication 1, caractérisé en ce que la concentration d'acide glycolique se monte à 40 à 60% en poids dans le diluat.

3. Procédé selon la revendication 1, caractérisé en ce que la tension de cellule appliquée pendant l'électrodialyse se monte à 1 à 2 V par paire de cellules.
